# EUROPEAN PATENT APPLICATION

(11) **EP 4 616 945 A1**
(43) Date of publication of application: **17.09.2025**
(21) Application number: 24173423.5
(22) Date of filing: 30.04.2024
(51) Int. Cl.: B01J 31/02, B01J 31/12, C07C 2/30

(54) **A CATALYST COMPOSITION FOR REDUCTION OF POLYMER FORMATION IN ETTHYLENE DIMERIZATION**

(30) Priority: 15.03.2024 IN 202441019311
(71) Applicant: Hindustan Petroleum Corporation Limited, Bengaluru 560067 (IN)
(72) Inventor: JAWOOR, Shailaja, 560067 Bangalore (IN); TALUKDAR, Monikangkana, 560067 Bangalore (IN); MOTE, Nilesh Rajesh, 560067 Bangalore (IN); PATIL, Yogesh Popatrao, 560067 Bangalore (IN); CHELLIAHN, Bennet, 560067 Bangalore (IN); SHESHACHALA, Srinivasa Narasimha, 560067 Bangalore (IN)
(74) Representative: V.O.

(57) **Abstract**

The present disclosure relates to a catalyst composition for reduction of polymer formation in ethylene dimerization comprising: a bio-based modifier as polymer suppressant for ethylene dimerization; a titanate compound; an alkyl aluminum compound; and an organic ether. The bio-based polymer suppressant is a compound selected from a group consisting of an isohexide derived ether compound of formula I, and an isomer of formula I or combination of thereof, wherein R₁ and R₂ are independently selected from a group consisting of CH₃, C₂H₅, n-C₃H₇, i-C₃H₇, n-C₄H₉, i-C₄H₉, Ph, C1 to C8 alkyl groups, an aryl group, and a heteroaryl group or combination of thereof.

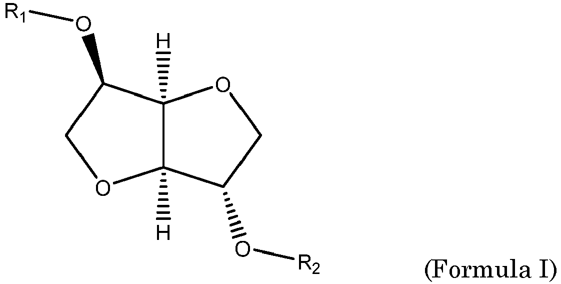

## Description

### Technical field

The present disclosure relates to a catalyst composition for reduction of polymer formation comprising: a bio-based modifier as polymer suppressant for ethylene dimerization; a titanate compound; an alkyl aluminum compound; and an organic ether.

### Background

The ethylene dimerization process leading to the production of 1-butene holds economic significance, especially when demanding high purity levels of 1-butene. As the primary member of linear 1-alkenes, 1-butene serves as a versatile chemical intermediate, playing a crucial role in the manufacturing of a diverse range of industrial products. Additionally, its varied applications enhance its importance in various industries. (1. McGuinness DS. Olefin oligomerization via metallacycles: Dimerization, trimerization, tetramerization, and beyond. Chem Rev 2011; 111:2321-2341*. 2.* Vogt D. Applied homogeneous catalysis with organometallic compounds. In: Cornils B, Herrmann WA, editors. vol. 1, Weinheim: Wiley-VCH Inc; 2002, p. 245*).* Typically, the ethylene dimerization process occurs in the liquid phase using a homogeneous catalytic system, such as Ti(OC₄H₉)₄-Al(C₂H₅)₃ along with an electron donor. Triethylaluminum (TEA), represented as Al(C₂H₅)₃, functions as an activator in this system. It facilitates the release of free coordination sites within the titanate complex, forming one or more Ti-C bonds by replacing its ethyl groups with the butoxide groups of the titanate complex. The catalyst modifiers, which are electron donor ligands, act as Lewis bases or polar organic compounds. When introduced into the catalyst system, they enhance the selectivity for the desired reaction. (Al-Sadoun AW. Dimerization of ethylene to 1-butene catalyzed by Ti(OR')4-AlR3. Appl Catal A 1993;105:1-40*.* Al-Jaralleh AM, Anabtawi JA, Siddiqui MAB, Aitani AM, Al-Sadoun AW. Ethylene dimerization and oligomerization to 1-butene and linear alpha-olefins: A review of catalytic system and processes. Catal. Today 1992; 14:1-121*).*

In the 1970s, the Institute of Problems of Chemical Physics, USSR Academy of Sciences, collaborated with various industrial institutions to pioneer the ethylene dimerization process yielding 1-butene. This marked the inception of the initial selective method for ethylene oligomerization. The progress of this development led to the establishment of two industrial plants dedicated to 1-butene production in the early 1980s. (S. S. Ivanchev, V. I. Zhukov, G. P. Belov, et al., Plast. Massy, No. 10, 82 (1990*)).*

WO2017/120310A1 discloses a catalyst system that reduces polymeric fouling in olefin oligomerization, in particular dimerization of ethylene to 1-butene, and comprises at least one titanate compound, at least one aluminum compound and one antifouling agent.

WO2019/060299A1 discloses a process for selectively producing 1-butene comprise combining at least one antifouling agent and at least one aluminum alkyl compound forms at least one antifouling agent in first step. The process further comprises feeding the antifouling feed stream, a catalyst comprising at least one titanate compound and ethylene into the reactor in the second step.

US11786889B2 discloses a catalyst system that may reduce polymeric fouling may include at least one titanate compound, at least one aluminum compound, and an antifouling agent. The antifouling agent may be chosen from one or more of a phosphonium or phosphonium salt; a sulfonate or a sulfonate salt; a sulfonium or sulfonium salt; an ester including a cyclic moiety; an anhydride; a polyether; and a long-chained amine-capped compound. The catalyst system may further include a non-polymeric ether compound.

Still there exists a need for development of a catalyst that addresses the problem associated with the formation of polymers during oligomerization reactions. Oligomerization systems face a recognized issue related to the creation of polymers. Extended periods of residence and insufficient heat dissipation from the intensely exothermic reactions result in the generation of residues primarily composed of polyethylene. The persistent fouling leads to more frequent interruptions in the process and elevated maintenance expenses for the removal of adhered polymer remnants. These polymer residues can accumulate layer by layer, potentially obstructing openings and ports in areas with fluid movement. Furthermore, a polymer coating on the reactor's wall may function as an insulator, impeding heat transfer within the reactor. The produced polymer also has the potential to negatively impact the reaction process by acting as a contaminant.

Thus, the present disclosure overcomes the polymer formation issues by using novel catalyst component, thereby reducing the operational downtime of plant.

### Objectives

The prime objective of the present disclosure is to develop the design and synthesis of a catalyst composition for selective dimerization of ethylene.

Another objective of the present disclosure is to develop the synthesis and characterization of isohexide based modifier as polymer suppressant for ethylene dimerization.

One more objective of the present disclosure is to develop a bio-based modifier as polymer suppressant for ethylene dimerization.

A further objective of the present disclosure is to overcome the polymer formation issues by using novel catalyst component, thereby reducing the operational downtime of plant.

Another objective of the present disclosure is to provide a process for synthesis of a bio-based modifier as polymer suppressant for ethylene dimerization.

A yet another objective of the present disclosure is to provide a catalyst composition that reduces polymer formation while maintaining relatively high dimerization activity.

Another objective of the present disclosure is to provide a process for producing 1-butene with high selectivity and conversion using a catalyst composition for reduction of polymer formation.

### Summary

The present disclosure relates to a catalyst composition for reduction of polymer formation comprising:
(a) a titanium alkoxide compound;
(b) an alkyl aluminum compound;
(c) an organic ether; and
(d) a bio-based polymer suppressant.

The present disclosure provides a process for synthesis of a bio-based modifier as polymer suppressant for ethylene dimerization.

In another aspect of the present disclosure provides a process for producing 1-butene with high selectivity and conversion using a catalyst composition for reduction of polymer formation comprising: a titanium alkoxide compound; an alkyl aluminum compound; an organic ether and a bio-based polymer suppressant; wherein the titanium alkoxide compound, alkyl aluminium compound, organic ether and bio-based polymer suppressant are present in a molar ratio in the range of 1 : 2-10 : 0.1-1 : 1-500.

These and other features, aspects, and advantages of the present subject matter will become better understood with reference to the following description. This summary is provided to introduce a selection of concepts in a simplified form. This summary is not intended to identify key features or essential features of the claimed subject matter, nor is it intended to be used to limit the scope of the claimed subject matter.

The titanium alkoxide compound is selected from a group consisting of titanium (IV) methoxide, Ti(OMe)₄, Titanium (IV) ethoxide, Ti(OEt)₄, Titanium(IV) i-propoxide Ti(i-OPr)₄, Titanium (IV) Butoxide, and Ti(OBu)₄ or combination of thereof. The alkyl aluminium compound is selected from a group of alkyl aluminium compounds consisting of tri-ethyl aluminium (TEAL), tri-isobutyl aluminium (TIBA), tri-isopropyl aluminium (TIPRA), tri-n-hexyl aluminium (TnHA), and diethyl aluminium chloride (DEAC) or combination of thereof. The organic ether is selected from a group consisting of tetrahydrofuran, tetrahydropyran, 1,4-dioxane, and 18-crown-6-ether or combination of thereof.

The bio-based polymer suppressant is a compound selected from a group consisting of an isohexide derived ether compound of formula I, and an isomer of formula I or combination of thereof; 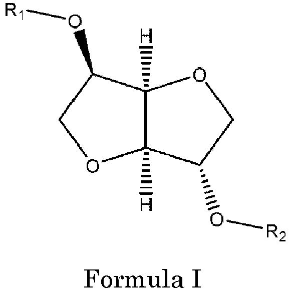 wherein R₁ and R₂ are independently selected from a group consisting of CH₃, C₂H₅, n-C₃H₇, i-C₃H₇, n-C₄H₉, i-C₄H₉, Ph, C1 to C8 alkyl groups, an aryl group, and a heteroaryl group or combination of thereof.

The synthesized modifiers are bio based prepared from isohexide which is derived from sorbitol. Isohexide based ether moieties have not been reported as a polymer suppressant for the ethylene oligomerization in particularly dimerization of ethylene to 1-butene process. The subsequent diethers of isohexides comprises ether moieties. The alteration of molecule and synthetic procedure outlined in the present disclosure is easy to carry out. The methodology provided in the present disclosure uses these isohexide derivatives as polymer suppressant components in the dimerization of ethylene to 1-butene.

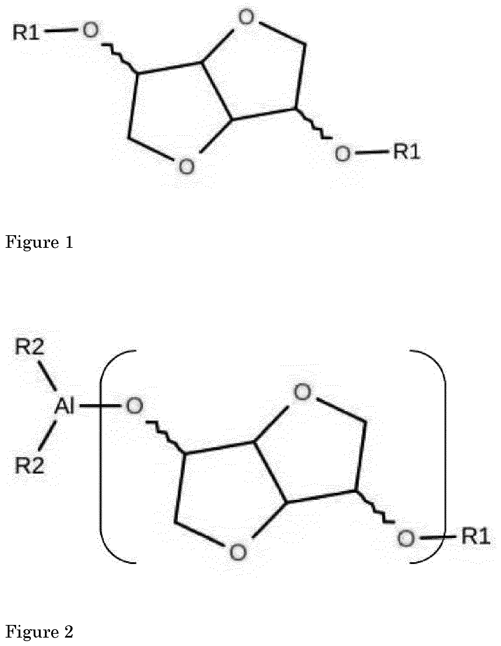

Figure 1 depicts the structure of the proposed bio-based modifier as a catalyst component as claimed in the present disclosure (R₁ = CH₃, C₂H₅, n-C₃H₇, i-C₃H₇, n-C₄H₉, i-C₄H₉, Ph).

Chemicals used in the synthesis of the catalyst composition as described in the present disclosure include Isosorbide, Sodium Hydride (NaH), DCM, water, Alkyl/Aryl Halides such as Methyl Iodide, Ethyl Iodide, etc., Titanium Alkoxides such as titanium (IV) methoxide, Ti(OMe)₄, Titanium (IV) ethoxide, Ti(OEt)₄, Titanium(IV) i-propoxide Ti(i-OPr)₄, Titanium (IV) Butoxide Ti(OBu)₄, hexane, toluene, heptane, methanol, hydrochloric acid, ethylene gas, alkyl aluminium such as triethylaluminium (TEAL), triisobutylaluminium (TIBA), triisopropyl aluminium (TIPRA), diethyl aluminium chloride (DEAC).

All the reactions were carried out using standard Schlenk technique and Glove box. The chemicals used for the catalyst preparations were stored under glove box having argon atmosphere. Dimerization reactions were performed using high pressure reactor. Hexane, heptane, toluene, cyclohexane was distilled over sodium benzophenone system and freshly distilled solvents were used throughout experiment.

Further, the present disclosure provides a process for producing 1-butene with high selectivity and conversion using a catalyst composition for reduction of polymer formation comprising: a titanium alkoxide compound; an alkyl aluminum compound; an organic ether and a bio-based polymer suppressant; wherein the titanium alkoxide compound, alkyl aluminium compound, organic ether and bio-based polymer suppressant are present in a molar ratio in the range of 1 : 2-10 : 0.1-1 : 1-500.

The synthesized bio-based polymer suppressant was characterized using the following techniques:
a) Fourier Transform Infrared Spectroscopy (FTIR): FTIR spectra were recorded with Perkin Elmer Spectrum GX equipment (Waltham, Massachusetts, USA). Samples were scanned with a resolution of 2 cm⁻¹ in the scan range of 4000-400 cm⁻¹.
b) NMR analysis: ¹H and ¹³C-NMR spectra were recorded on Bruker Avance 500 MHz spectrometer. Deuterated solvents for NMR experiments were obtained from Aldrich Chemical Co.
c) Gas Chromatography: Gas samples were analyzed by using Perkin Elmer Clarus 690 with elite alumina column having 0.53 mm ID and 30-meter length. For liquid samples, GC-VUV, Rxi-1ms column having 30-meter length and 0.25 mm ID.

### Synthesis of Modifier for Polymer Suppressant

*Synthetic protocol:* In a clean 250 ml Schlenk RBF, NaH (2.05 g, 0.0855 mol) was taken. To this, 100 ml of THF and 1 equivalent of isosorbide was added and taken the RB temperature to 0-5 °C. Added 2 equivalent of Alkyl/aryl halide at the same temperature. Once the addition was over, the reaction mixture was stirred for 12-24 h. Workup was done with ice water and extracted with DCM. The desired polymer suppressant yield obtained is > 90 %.

The resultant molecule was established using ¹H, ¹³C-NMR and FTIR analysis. A plausible chemical structure of polymer suppressant (R₁, R₂ = CH₃, C₂H₅, n-C₃H₇, i-C₃H₇, n-C₄H₉, i-C₄H₉, Ph) is illustrated in Figure 2.

The present disclosure is further illustrated by reference to the following examples which is for illustrative purposes only and does not limit the scope of the disclosure in any way. These examples are not intended to be inclusive of all aspects of the subject matter disclosed herein, but rather to illustrate representative features, methods, compositions, and results. These examples are not intended to exclude equivalents and variations of the present disclosure, which are apparent to one skilled in the art.

### Example 1

The oligomerization experiments were conducted in a 1 L High Pressure Reactor. Prior to the experimental runs, the reactor was subjected to an inertization process which included evacuating the reactor with vacuum pump and heating to 160 °C. After a stable temperature had been reached, the reactor was pressurized to 4 bar with nitrogen. Then, three minutes following the start of the pressurization, the gas outlet valves were opened to release the nitrogen to the exhaust. Two minutes after the gas release had started, the valves from the main exhaust pipe to the vacuum pump were opened to evacuate the reactor. The reactor was evacuated for 15 minutes. The gas outlet valves were then closed, and the reactor was pressurized with nitrogen again. The pump-pressurize cycles were run for at least 2-3 hours. The reactor was then evacuated in a vacuum for a further 2-3 hours. During the last one hour, the reactor was cooled down to 40 °C. The reactor was then pressurized to 1-3 bar until the reaction was started.

Stock solutions were prepared, which included the components of the catalyst mixtures. The two stock solutions were prepared in a glove box. Heptane was utilized as a solvent. The reactor was filled with 80 % of heptane. The first solution contained the TEAL co-catalyst mixed with polymer suppressant and 10 % of the heptane. The second solution contained the titanium tetrabutoxide catalyst, the THF, and mixed with 10 % of the heptane. The first solution and the second solution were put into the reactor, respectively under positive ethylene flow. Then, the reactor was set to the desired pressure. The temperature in the reactor rose and the temperature was set to the target value of 55 °C. After the start of the ethene dosage, the reaction was run for 30 min. After 30 minutes of reaction time, the reaction was terminated by the injection of 1 mL of methanol. The pressure was released from the reactors, and the temperature was set to 25 °C.

The residue in the reactor was then washed with a 10 wt.% aqueous hydrochloric acid solution to dissolve any catalyst residues. The remaining solid polymer was filtered and dried overnight in an oven at 110 °C and weighed.

### Example II

To evaluate the polymer suppressant effects of the catalyst composition as described in the present disclosure, ethylene oligomerization reactions were carried out and evaluated. The control sample which had no polymer suppressant is listed as "Comparative Example" in Table 1. For the experiments, catalyst mixtures were used that contained titanium tetrabutoxide (denoted as "Ti" in Table 2), THF, triethyl aluminum (referred to as "TEAL" in Table 2), and polymer suppressant (denoted as "PS" in Table 2). The PS concentrated is listed in Table 1. The molar ratio of Ti : THF : TEAL in the examples was 1 : 0.8 : 8.

### Comparative Example 1

The process for oligomerization as outlined in Example I with all the same parameters is followed except with the catalyst composition as Ti / TEAL / THF in the molar ratio of is 1 : 0.8 : 8 and at a pressure 23.5 bar. The value for 1-butene selectivity for reactions with the catalyst composition of comparative example 1 is mentioned in Table 1.

### Example 1

The process for oligomerization as outlined in Example I with all the same parameters is followed except with the catalyst composition as Ti / TEAL / THF / PS-1 and at a pressure 23.5 bar. The molar ratio of catalyst composition Ti : THF : TEAL is 1 : 0.8 : 8. The polymer suppressant PS-1 is present in a concentration of 1500 ppm and is 1,4:3,6-Dianhydro-2,5-di-O-methyl-D-glucitol. Table 1 mentions the experimental data of ethylene dimerization to 1-butene including 1-butene selectivity.

### Example 2

The process for oligomerization as outlined in Example I with all the same parameters is followed except with the catalyst composition as Ti / TEAL / THF / PS-2 and at a pressure 23.5 bar. The polymer suppressant PS-2 which is present in a concentration of 1500 ppm and is 1,4:3,6-Dianhydro-2,5-di-O-ethyl-D-glucitol. The experimental data of ethylene dimerization to 1-butene including 1-butene selectivity is provided in Table 1.

### Example 3

The process for oligomerization as outlined in Example I with all the parameters being same except with the catalyst composition as Ti / TEAL / THF / PS-3 and at a pressure of 23.5 bar is carried out. The polymer suppressant PS-3 is present in a concentration of 1500 ppm and is 1,4:3,6-Dianhydro-2,5-di-O-isoproyl-D-glucitol. Table 1 mentions the experimental data of ethylene dimerization to 1-butene including 1-butene selectivity.

### Comparative Example 2

All the parameters in this example are the same as those mentioned in comparative example 1 except for the process for oligomerization being carried out at a pressure of 12.5 bar. Table 2 shows the dimerization activity and % polymer reduction for reactions for the catalyst composition described by comparative example 2.

### Example 4

All the parameters in this example are the same as those mentioned in Example 1 except for the process for oligomerization being carried out at a pressure of 12.5 bar. Table 2 shows the dimerization activity and % polymer reduction for reactions for the catalyst composition described by Example 4.

### Example 5

All the parameters in this example are the same as those mentioned in Example 2 except for the process for oligomerization being carried out at a pressure of 12.5 bar. Table 2 shows the dimerization activity and % polymer reduction for reactions for the catalyst composition described by Example 5.

### Example 6

All the parameters in this example are same as those mentioned in Example 3 except for the process for oligomerization being carried out at a pressure of 12.5 bar. Table 2 shows the dimerization activity and % polymer reduction for reactions for the catalyst composition described by Example 6.

Table 1 shows the conversion, 1-butene selectivity for reactions which utilized each of the sample catalyst composition. It is evident from Table 1 that the conversion, 1-butene yield and selectivity increases with addition of the polymer suppressant.

The Reaction Conditions for ethylene dimerization to 1-butene for the data mentioned in Table 1 are as follows:
Catalyst concentration = 200 mg; Temperature = 50-60 oC; Time = 0.5 h, PS concentration = 1500 ppm;
Pressure: Comparative Example 1 = 23.5 bar & Comparative Example 2 = 12.5 bar.
Pressure: Example 1 = 23.5 bar & Example 2 = 12.5 bar.

**Table 1: Experimental data of ethylene dimerization to 1-butene**

| Examples | % Conversion | 1-Butene Yield | % C4 Selectivity | % 1-Butene-Selectivity | % C6+ Selectivity |
|---|---|---|---|---|---|
| Comparative Example 1 | 79.3 | 79.3 | 98.1 | 99.5 | C6 = 1.9 % |
| Example 1 | 96.6 | 96.3 | 99.6 | 99.7 | C6 = 0.3 % |
| Comparative Example 2 | 88.6 | 88.4 | 99.4 | 99.6 | C6 = 0.6 % |
| Example 2 | 94.3 | 93.59 | 98.7 | 99.6 | C6 = 1.50 % |

### Example III

Table 2 shows the dimerization activity and % polymer reduction for reactions which utilized each of the sample catalyst. As is evident by the reaction data of Table 2, the addition of the polymer suppressant reduced polymer formation to some degree while maintaining relatively high dimerization activity.

For the experiments, catalyst mixtures were used that contained titanium tetrabutoxide (denoted as "Ti" in Table 2), THF, triethyl aluminum (referred to as "TEAL" in Table 2), and polymer suppressant (denoted as "PS" in Table 2). The PS concentrated is listed in Table 1. The molar ratio of Ti : THF : TEAL in the examples was 1 : 0.8 : 8.

**Table 2: Dimerization activity and % polymer reduction for Oligomerization reactions**

| Sl. No. | Composition of Catalyst | Activity (grams of reacted C2/mmoles of Ti catalyst/hour) | % Polymer reduction | Polymer Suppressant (PS) |
|---|---|---|---|---|
| Comparative Example 1 | Ti/TEAL/THF | 256 | - | Nil |
| Example 1 | Ti/TEAL/THF/PS-1 | 306 | 68 | PS-1 |
| Example 2 | Ti/TEAL/THF/PS-2 | 269 | 28 | PS-2 |
| Example 3 | Ti/TEAL/THF/PS-3 | 264 | 24 | PS-3 |
| Comparative Example 2 | Ti/TEAL/THF | 180 | - | Nil |
| Example 4 | Ti/TEAL/THF/PS-1 | 251 | 48 | PS-1 |
| Example 5 | Ti/TEAL/THF/PS-2 | 213 | 27 | PS-2 |
| Example 6 | Ti/TEAL/THF/PS-3 | 190 | 21 | PS-3 |

The Reaction Conditions for dimerization activity and % polymer reduction for Oligomerization reactions for the data mentioned in Table 2 are as follows:
Temperaure = 50-60 oC; Time = 0.5 h;
PS concentration = 1500 ppm
PS-1: 1,4:3,6-Dianhydro-2,5-di-O-methyl-D-glucitol
PS-2: 1,4:3,6-Dianhydro-2,5-di-O-ethyl-D-glucitol
PS-3: 1,4:3,6-Dianhydro-2,5-di-O-isoproyl-D-glucitol

### Advantages:

The present disclosure provides a catalyst composition for reduction of polymer formation.

The present disclosure provides a catalyst composition exhibiting high selectivity and conversion for producing 1-butene from ethylene dimerization reaction.

The present disclosure provides a bio-based modifier as a polymer suppressant, thus offering an environmentally friendly catalyst composition.

Although the subject matter has been described in considerable detail with reference to certain preferred embodiments thereof, other embodiments are possible. As such, the spirit and scope of the subject matter should not be limited to the description of the preferred embodiment contained therein.

## Claims

1. A catalyst composition for reduction of polymer formation in ethylene dimerization comprising:
a) a titanium alkoxide compound;
b) an alkyl aluminum compound;
c) an organic ether; and
d) a bio-based polymer suppressant.

2. The catalyst as claimed in claim 1, wherein titanium alkoxide compound to alkyl aluminum compound is present in a molar ratio in the range of 1 : 2-10.

3. The catalyst as claimed in claim 1, wherein titanium alkoxide compound to organic ether is present in a molar ratio in the range of 1 : 0.1-1.

4. The catalyst as claimed in claim 1, wherein titanium alkoxide compound to bio-based polymer suppressant is present in a molar ratio in the range of 1 : 1-500.

5. The catalyst as claimed in claim 1, wherein the titanium alkoxide compound is selected from a group consisting of titanium (IV) methoxide, Ti(OMe)₄, Titanium (IV) ethoxide, Ti(OEt)₄, Titanium(IV) i-propoxide Ti(i-OPr)₄, Titanium (IV) Butoxide, and Ti(OBu)₄ or combination of thereof.

6. The catalyst as claimed in claim 1, wherein the alkyl aluminium compound is selected from a group of alkyl aluminium compounds consisting of tri-ethyl aluminium (TEAL), tri-isobutyl aluminium (TIBA), tri-isopropyl aluminium (TIPRA), tri-n-hexyl aluminium (TₙHA), and diethyl aluminium chloride (DEAC) or combination of thereof.

7. The catalyst as claimed in claim 1, wherein the organic ether is selected from a group consisting of tetrahydrofuran, tetrahydropyran, 1,4-dioxane, and 18-crown-6-ether or combination of thereof.

8. The catalyst as claimed in claim 1, wherein the bio-based polymer suppressant is a compound selected from a group consisting of an isohexide derived ether compound of formula I, and an isomer of formula I or combination of thereof; 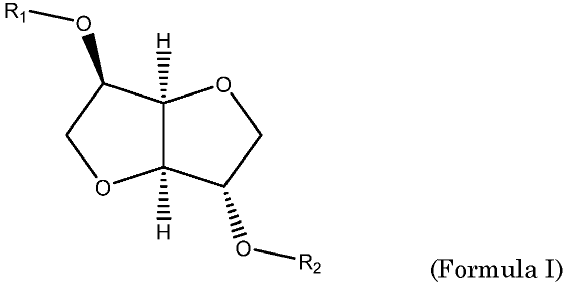 wherein R₁ and R₂ are independently selected from a group consisting of CH₃, C₂H₅, n-C₃H₇, i-C₃H₇, n-C₄H₉, i-C₄H₉, Ph, C1 to C8 alkyl groups, an aryl group, and a heteroaryl group or combination of thereof.

9. A process for producing 1-butene with high selectivity and conversion from a catalyst composition for reduction of polymer formation comprising: a titanium alkoxide compound; an alkyl aluminum compound; an organic ether and a bio-based polymer suppressant.

10. The process as claimed in claim 9, wherein catalyst composition for reduction of polymer formation in ethylene dimerization comprises the titanium alkoxide compound, alkyl aluminium compound, organic ether and bio-based polymer suppressant present in a molar ratio in the range of 1 : 2-10 : 0.1-1 : 1-500.

## Amended claims

### Amended claims in accordance with Rule 137(2) EPC.

1. A catalyst composition for reduction of polymer formation in ethylene dimerization comprising:
a) a titanium alkoxide compound;
b) an alkyl aluminum compound;
c) an organic ether; and
d) a bio-based polymer suppressant, wherein the bio-based polymer suppressant is a compound selected from a group consisting of an isohexide derived ether compound of formula I, an isomer of formula I, or combination of thereof; 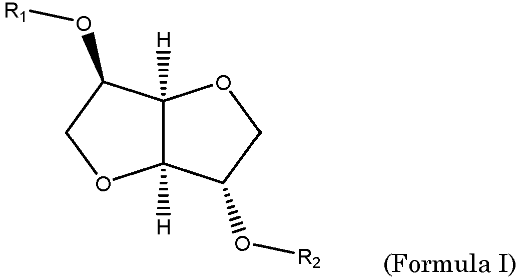 wherein R₁ and R₂ are independently selected from a group consisting of CH₃, C₂H₅, *n*-C₃H₇, *i̅-*C₃H₇, *n*-C₄H₉, *i*-C₄H₉, Ph, C1 to C8 alkyl groups, an aryl group, and a heteroaryl group, or combination of thereof.

2. The catalyst composition as claimed in claim 1, wherein the titanium alkoxide compound to the alkyl aluminum compound is present in a molar ratio in a range of 1 : 2-10.

3. The catalyst composition as claimed in claim 1, wherein the titanium alkoxide compound to the organic ether is present in a molar ratio in a range of 1 : 0.1-1.

4. The catalyst composition as claimed in claim 1, wherein the titanium alkoxide compound to the bio-based polymer suppressant is present in a molar ratio in a range of 1 : 1-500.

5. The catalyst composition as claimed in claim 1, wherein the titanium alkoxide compound is selected from a group consisting of titanium (IV) methoxide (Ti(OMe)₄), Titanium (IV) ethoxide (Ti(OEt)₄), Titanium(IV) *i*-propoxide (Ti(*i*-OPr)₄), Titanium (IV) Butoxide (Ti(OBu)₄), or a combination thereof.

6. The catalyst composition as claimed in claim 1, wherein the alkyl aluminium compound is selected from a group of alkyl aluminium compounds consisting of tri-ethyl aluminium (TEAL), tri-isobutyl aluminium (TIBA), tri-isopropyl aluminium (TIPRA), tri-n-hexyl aluminium (TₙHA), diethyl aluminium chloride (DEAC), or a combination thereof.

7. The catalyst composition as claimed in claim 1, wherein the organic ether is selected from a group consisting of tetrahydrofuran, tetrahydropyran, 1,4-dioxane, 18-crown-6-ether, or a combination thereof.

8. A process for producing 1-butene with high selectivity and conversion from the catalyst composition for reduction of polymer formation as claimed in claim 1.

9. The process as claimed in claim 8, wherein the catalyst composition for reduction of polymer formation in ethylene dimerization comprises the titanium alkoxide compound, the alkyl aluminium compound, the organic ether and the bio-based polymer suppressant in a molar ratio in a range of 1 : 2-10 : 0.1-1 : 1-500.
